# EUROPEAN PATENT SPECIFICATION

(11) **EP 2 959 853 B1**
(45) Date of publication and mention of the grant of the patent: **14.02.2018**
(21) Application number: 14754828.3
(22) Date of filing: 21.02.2014
(51) Int. Cl.: A61B 17/221

(54) **THROMBECTOMY DEVICE AND THROMBECTOMY EQUIPMENT**
THROMBEKTOMIEVORRICHTUNG UND AUSRÜSTUNG FÜR THROMBEKTOMIE
DISPOSITIF DE THROMBECTOMIE ET ÉQUIPEMENT DE THROMBECTOMIE

(30) Priority: 21.02.2013 CN 201310056463
(43) Date of publication of application: 30.12.2015
(73) Proprietor: MicroPort NeuroTech (Shanghai) Co., Ltd., Shanghai 201318 (CN)
(72) Inventor: XU, Qingshun, Shanghai 201318 (CN); NI, Zunzhang, Shanghai 201318 (CN); JIN, Qiaorong, Shanghai 201318 (CN); XIE, Zhiyong, Shanghai 201318 (CN); JI, Jie, Shanghai 201318 (CN); FAN, Yinping, Shanghai 201318 (CN); ZHANG, Yu, Shanghai 201318 (CN); LUO, Qiyi, Shanghai 201203 (CN)
(74) Representative: Lavoix
(86) International application number: PCT/CN2014/072387
(87) International publication number: WO 2014/127738

(56) References cited:
- WO-A1-2011/144336
- WO-A2-2012/120490
- CA-A1- 2 737 653
- CN-A- 101 027 004
- CN-A- 101 088 472
- CN-A- 102 355 871
- CN-A- 102 596 098
- US-A1- 2006 058 838
- US-A1- 2009 306 678
- US-A1- 2011 009 941
- US-A1- 2011 098 738
- US-A1- 2012 209 311
- US-B2- 7 063 707

## Description

### TECHNICAL FIELD

The present invention relates to medical instruments and, in particular, to a medical instrument for vascular intervention and, more particularly, to a means for removing a thrombus from an intracranial vessel. The invention is also directed to a thrombus removal device incorporating the means for removing the thrombus, which is particularly suited to the removal of a thrombus from an intracranial vessel.

### BACKGROUND

Intravascular thrombosis is a high-incidence disease frequently encountered in clinical practice, and intracranial thrombosis has the most serious consequences and can lead to cerebral infarction. Intracranial thrombosis is known to have high incidence, morbidity, mortality and recurrence rate, and is ranked as a leading cause of disability and death among middle-aged and elderly people. With the increasing elderly population and living standards, the number of patients with cerebrovascular diseases has been increasing. Surveys reveal that, in China, three million people are initially diagnosed with cerebral thrombosis every year and acute strokes have accounted for 81.6% of all strokes, compared to the previous percentage of 55.8%, becoming the most frequent disease of the brain.

Blood flow restoration is crucial for the treatment of acute ischemic strokes. At present, the treatment of intracranial thrombosis is based essentially on lysis by thrombolytic drugs and mechanical removal.

The lysis approach involves infusing a thrombolytic agent around an occlusive thrombus in a blood vessel to instantly create there a high concentration of the agent to facilitate lysis of the thrombus and increase the likelihood of blood flow restoration. However, according to findings of the U.S. National Institute of Neurological Disorders and Stroke (NINDS), intravenous lysis should be effectuated within 3 hours since the onset, and the time window for intra-arterial lysis is only 6 hours. Such limited time windows narrow the application of this treatment only to small clots and make it unfavorable for relatively large clots. As a result, only about 3-5% of patients are suitable for the lysis approach.

Mechanical removal is to deliver a thrombus removal device to a lesion, which helps to retrieve a clot by means of a sheath. Mechanical removal may be accomplished in several ways such as, for example, capture, extraction, rotary cutting, etc. Although the capturing approach performs best in removing large clots, it tends to cause plaques to dislodge from the vessel wall or cause clot fragments, which may then travel with the blood flow and block a downstream vessel. If the blocked vessel happens to be an important branch vessel, a severe medical accident may occur. In addition, use of the capturing approach is also associated with the issue of incomplete removal and remaining thrombus material, which often lead to secondary acute embolism. The extraction approach is more suitable for the removal of relatively small clots. When used to remove a large clot, it requires fragmentation of the large clot by repeatedly disturbing it beforehand in order to avoid occlusion of the used extracting catheter. The extraction process is complex and is very likely to lead to vascular injury. The rotary cutting approach can lead to larger damage to treated vessels and tends to cause complications.

Chinese Utility Model Application No. 03210077.9 disclosed an ellipsoidal distal protection device, comprising a stented ellipsoidal main body tapered at the both ends and a porous polymer membrane covering an upper portion of the stent. The membrane allows the passage of normal blood flow and traps solid particles such as dislodging plaques or emboli, thereby protecting downstream vessel from being occluded. Chinese Utility Model Application No. 200620164685.4 disclosed a thrombus removal device for use in lower limb arteries, consisting of an umbrella portion, a main shaft, a pusher guide wire, an inner catheter, an outer sheath and a core in the outer sheath. The umbrella portion is made up of shape memory alloy wires, two long struts and one short strut, and is angularly soldered to the main shaft. Additionally, a mesh structure for trapping emboli is disposed around the umbrella portion. U.S. Patent Publication Nos. 2007/0005103, 2008/0243170 and 2011/0098738 disclosed emboli capturing devices with the similar structures, each comprising a stent and a filter mesh or membrane for trapping emboli that is attached to the stent. All of these devices, however, are too bulky to be delivered to an intracranial vessel.

Chinese Patent Application No. 01114889.6 disclosed a reusable temporary emboli filter which can be used in intracranial vessels. The emboli filter is comprised of a filter body, a guide tip, a guide pole, an interconnection and an outer sheath. The filter body is braided from shape memory alloy wires. U.S. Patent Publication No. 2011/0160763 disclosed a thrombus removal device also useable in intracranial vessels. This thrombus removal device includes a scaffold for capturing a thrombus, which can be braided from shape memory alloy wires. However, these thrombus removal devices are both structurally complicated and lack of stiffness for holding a captured thrombus or embolus during the retrieval process. As a result, it is possible for the captured thrombus or embolus to dislodge during the retrieval process.

Furthermore, use of the conventional thrombus removal devices tends to cause distal embolization due to possible dislodgement of plaques from the vessel wall or formation of clot fragments during the use. It has been reported that fragmented pieces and pellets of blood clots travelling toward distal vessels, which expose the patients to an increased risk of vascular reocclusion, are witnessed in about 70% of endovascular thrombus removal procedures. For example, the Solitaire™ FR system, available from the U.S. EV3 Inc., is an intracranial thrombus removal device that is most accepted among those commercially available by the western physicians in the treatment of acute thrombosis. The system employs a capturing approach, in which it captures a thrombus after being delivered over a hypotube and being deployed therefrom and is then retrieved from the body together with the thrombus in a state of being received together therewith in a guide catheter having an larger inner diameter. According to the physicians, as the thrombus removal device is inadequate in radial force, it is often the case that the captured thrombus detaches from the thrombus removal device during the retrieval process, thus requiring repeated recaptures. This leads to increased suffering of the patient, extended operation time, and increased likelihood of distal embolization.

U.S. Patent Publication No. US2006/058838A1 discloses a thromboembolic removal system for treating ischemic stroke, including a guide and occlusion catheter, a delivery and aspiration catheter, an aspiration pump, a thromboembolic receiver, and a thromboembolic separator.

U.S. Patent Publication No. US2009/306678A1 discloses a retrieval device for entrapping and retaining a foreign object located in a body vessel for its extraction therefrom. The device includes a cage having a proximal end and a distal end and a longitudinal axis extending between the proximal and distal ends. The cage is radially expandable about the longitudinal axis to define an expanded state and to form an opening at the distal end for receiving the foreign object. The cage is collapsible to define a collapsed state and to retain the foreign object therein such that the foreign object is substantially aligned with the longitudinal axis. Extending proximally from the proximal end of the cage is a cable for retrieval of the device in the collapsed state.

WO2011144336A1 discloses a medical device for removing concretions from hollow organs of the body, with a functional element, which has a rotationally symmetrical lattice structure and a means for holding a concretion in the lattice structure, and with a catheter for introducing the functional element into the body and for removing it therefrom, wherein the functional element can be converted from a compressed state in the catheter to an expanded state outside the catheter, in which expanded state the functional element is arranged distally from the catheter. The invention is characterized in that the lattice structure has a cutting area with webs, which are adapted in such a way that they at least partially pass radially through the concretion when the functional element is converted from the compressed state to the expanded state, and the holding means includes at least one holding element, which is connected on the one hand to the lattice structure and on the other hand to a guide wire arranged in the catheter, in such a way that the holding element is deflected radially inwards in the expanded state of the functional element.

U.S. Patent No. US2011009941A1 discloses a device including a self-expandable member having a proximal end portion and a main body portion. Each of the self-expandable portions consists of a plurality of cell structures formed by intersecting strut members. At least one proximal cell structure in the proximal end portion has one or more struts that have a width and/or thickness greater than the width and/or thickness of the majority of strut members in the main body of the expandable member. At least some of the intersecting strut members have a thickness to width ratio of greater than one. Attached to at least one of the proximal-most cell structures in the proximal end portion is a proximally extending flexible wire having sufficient length and flexibility to navigate the tortuous vasculature of a patient. The device is delivered to the treatment site through the lumen of a delivery catheter.

WO2012/120490A2 discloses a clot retrieval device for removing occlusive clot from a blood vessel which includes an inner elongate body and an outer elongate body at least partially overlying the inner elongate body. The device also includes an elongate member or shaft having a proximal end which extends exterior of a patient so that a user can retrieve the stent-basket device and captured clot by retracting the shaft. The outer elongate body and the inner elongate body are connected to the distal end of the shaft and are expandable relative to the shaft from a collapsed delivery configuration to an expanded deployed configuration. The outer elongate body is expandable relative to the inner elongate body to a radial extent which is greater than the radial extent of the inner body in the deployed configuration. The stent-basket construction of the device creates a reception space between the inner and outer to receive the target clot. The outer body is configured to allow as much as possible of the clot to migrate through it into the internal reception space. Housing the clot in this reception space rather than pinning it to the wall of the vessel means that the clot is under less compression and can thus be retracted at a lower force. The inner elongate body and a distal capture net protects the distal vascular bed from embolisation.

U.S. Patent No. US7063707B2 discloses alternative designs, materials and manufacturing methods for medical retrieval devices. Some embodiments pertain to a medical retrieval device including a cage having internal migration barriers therein. Some embodiments pertain to a medical retrieval device including a cage having variable diameters along the length thereof. Several alternative medical retrieval devices constructions and/or designs including methods and techniques of construction are also disclosed.

CA2737653A1 discloses apparatus and methods which are provided for removing obstructive material within a body lumen. The apparatus includes a macerator device deployable from a sheath that includes an expandable cage carried by a shaft and within a constraint tube. The shaft is movable relative to the constraint tube for deploying and expanding the cage within a body lumen such that an open end of the cage is oriented towards obstructive material. The cage is advanced to capture the material or the material is directed into the cage using an expandable member expanded beyond the material and retracted to direct the material into the cage. The cage is withdrawn into the constraint tube to compress the cage radially inwardly. Material extending through apertures in the cage are sheared off by a sharpened edge of the constraint tube. The smaller, sheared off particles are then aspirated from the body lumen through the sheath.

Therefore, there is a need for an improved thrombus removal device, which 1) entails a simple and convenient thrombus removal process; 2) provides a high flexibility, high capturing ability and high radial force while causing no vascular injury; and 3) more firmly holds the captured thrombus with less likelihood of dislodgement during the retrieval.

### SUMMARY OF THE INVENTION

In view of this, the present invention addresses the issue of distal re-embolization caused by the vessel wall plaques or clot fragments dislodging from the conventional thrombus removal devices by presenting an improved thrombus removal system with the additional benefit of a small profile and high flexibility. The invention is disclosed in independent claim 1 and preferred embodiments are disclosed in the dependent claims. According to one aspect, the present invention provides a means for removing a thrombus from a vessel, which is a mesh structure defining a lumen and is switchable between a contracted configuration and an expanded configuration. The thrombus removal means has a plurality of inwardly arching ribs extending from the mesh structure into the lumen, each of the plurality of inwardly arching ribs having a three-dimensional contour and both ends thereof fixed to the mesh structure, the plurality of inwardly arching ribs are oriented in an axial direction of the mesh structure, wherein a distance h1 from a location where one of the plurality of inwardly arching ribs (321) is fixed to the mesh structure to a position where a corresponding cell of the mesh structure is interconnected with an adjacent cell thereof in a widthwise direction of the corresponding cell satisfies: 1/4h<h1<3/4h, and/or a distance h2 from a middle point of the one of the plurality of inwardly arching ribs (321) to the position where the corresponding cell is interconnected with the adjacent cell satisfies: 3/4h<h2<7/4h, wherein h is half of a width of the corresponding cell. As used herein, the term "three-dimensional contour" denotes a non-planar contour. This results in an increased thrombus capturing ability, thereby facilitating the likelihood of capture in a single pass. This can reduce the suffering of a patient during the operative procedure and allows a captured thrombus to be firmly held. As a result, dislodgement of the captured thrombus during the retrieval which may cause secondary embolization in distal small vessels is prevented, and the likelihood of complications occurring due to the removal is reduced.

The mesh structure of the thrombus removal means can be formed by, for example, laser-cutting a nitinol tube. The plurality of inwardly arching ribs may be formed by heat treatment, and form an integrated structure together with the mesh structure.

According to the present invention, when the number of the plurality of inwardly arching ribs is a predetermined number, the plurality of inwardly arching ribs are preferably oriented in the axial direction. Axially oriented ribs can result in an adequately lower radial force, by about 30% than that when they are circumferentially oriented. In addition, the axially oriented ribs allow the thrombus removal means to have a smaller profile when in the contracted configuration. This enables the thrombus removal means to be delivered with a smaller force and to reach smaller blocked vessel. Compared to the case of circumferentially oriented ribs, the axially oriented ribs can result in a reduction of about 50% of the required delivery force.

Preferably, each of the inwardly arching ribs forms an angle of 30-90° with a tangent of an outer circumference of the mesh structure at a position where the inwardly arching rib is fixed to the mesh structure. The angles of the plurality of inwardly arching ribs may be identical or not identical. For example, the angles may gradually increase, decrease or periodically vary along a direction from a proximal end toward a distal end of the thrombus removal means.

Preferably, the thrombus removal means has an overall cylindrical shape in the expanded configuration. This imparts desirable radial force to the thrombus removal means, so that it can well adhere to the wall of a vessel after being deployed from an outer sheath of a thrombus removal device.

Preferably, in the expanded configuration, the thrombus removal means has a nearly oval opening. This allows the thrombus removal means to be easily crimped into the outer sheath of the device while ensuring a sufficient area of the thrombus removal means for thrombus capture.

According to the present invention, the plurality of inwardly arching ribs may have one of the following distribution patterns: spiraling toward the same direction, spiraling toward opposite directions, spiraling toward intersecting directions, being distributed in a symmetrical manner, and being distributed in an alternating manner. Additionally, the plurality of inwardly arching ribs may be located at the same or different positions with respect to the corresponding cells of the mesh structure and may extend inwardly the same or different lengths. Adjusting such positions and lengths of the plurality of inwardly arching ribs can result in different radial force and thrombus capturing abilities of the thrombus removal means, which are adapted to thrombi with different diameters and hardness.

Preferably, cells of the mesh structure have a length-to-width ratio greater than 1 and smaller than 2.

According to the present invention, the thrombus removal means may be fully or partially closed at the distal end, and is preferably fully closed thereat. This effectively prevents a small thrombus that has been captured by the thrombus removal means from dislodging from the distal end during the retrieval. The closure may be accomplished by a soldering joint, a metal sleeve or a metal wire coil. The sleeve or coil may be formed of platinum, gold, tungsten, platinum-tungsten, platinum-iridium, etc. and may function as a radiopaque marker.

Preferably, at the distal end, the thrombus removal means may terminate at an elongate tip which is wound with a coil. The combined use of the elongate tip and the coil can increase the flexibility at the distal end of the thrombus removal means, which makes the thrombus removal means better compliant with and less stimulatory to the wall of a vessel, in particular, a tortuous vessel, thus preventing puncture of the vessel wall by the thrombus removal means which may lead to a serious accident. The coil may be formed of a platinum, platinum-iridium, silver, etc. wire.

According to another aspect, the present invention provides a thrombus removal device, which includes a guide wire, a pusher shaft, an outer sheath and an inner tube movable relative to the outer sheath. The guide wire is inserted through the inner tube which is in turn received in the outer sheath and is movable over the guide wire. The thrombus removal device further includes a thrombus removal means as defined above. The thrombus removal means is attached to the pusher shaft (e.g., by soldering, riveting and press fitting) and is crimped into the inner tube together with the pusher shaft after they are assembled in this manner. In addition, the thrombus removal means is switchable between a contracted configuration and an expanded configuration upon being retracted or advanced by the pusher shaft such that it is in the contracted configuration when received in the inner tube and is pushed out of the inner tube when in the expanded configuration. Preferably, the outer sheath further includes an occlusion balloon disposed at its distal end. This thrombus removal device is capable of firmly holding a captured thrombus with an improved stiffness and with no possibility for the thrombus to dislodge therefrom.

Preferably, the thrombus removal means is attached to the pusher shaft by means of a tethering connector which is positioned closely adjacent to a vessel wall when the thrombus removal means is in the expanded configuration. This significantly facilitates the capture of a large clot. The tethering connector may be effectuated as a soldering joint, a metal sleeve or a metal wire coil. The sleeve or coil may be formed of platinum, gold, tungsten, platinum-tungsten, platinum-iridium, etc. and may function as a radiopaque marker.

Preferably, the pusher shaft has a radius decreasing stepwise from a proximal end of the pusher shaft toward a distal end thereof. The reduced profile at the distal end, as well as, the gradually shrinking radial dimension along the axial direction can provide a further increase in the flexibility. The pusher shaft may be fabricated from, for example, nitinol or stainless steel, and can assume a generally cylindrical shape.

Preferably, a distal portion of the pusher shaft is wound with a coil which may be made of a platinum, platinum-iridium, silver, etc. wire. The coil disposed over the distal portion can result in an increase in torque resistance. Additionally, proper design of coil length and push shaft sections can achieve a synergistic effect, i.e., increasing torque resistance while not significantly reducing flexibility. Therefore, use of the coil can not only augment the delivery ability of the pusher shaft, but can also enhance its visibility in X-rays. This can result in an improvement in positioning accuracy.

Preferably, a heat shrink tube is disposed at least over the coil. Materials from which the heat shrink tube can be fabricated include polytetrafluoroethylene (PTEF), polyvinylchloride (PVC), polyethylene terephthalate (PET) and fluorinated ethylene propylene (FEP). Use of the heat shrink tube can enhance force transfer when the push shaft is advancing in a vessel.

According to a further aspect which is not part of the invention, there is provided use of a thrombus removal device for removing a thrombus from an intracranial vessel having an occlusive thrombus. In the use, an angiographic assessment may be first performed to identify the vessel. The outer sheath may then be delivered around the thrombus, followed by inserting the guide wire through the thrombus. The inner tube may then be delivered over the guide wire until it passes through the thrombus. After the positioning, the pusher shaft may be held stationary and the inner tube may be retracted concurrently to cause the thrombus removal means to expand within the thrombus. After that, the occlusion balloon on the outer sheath may be caused to occlude the vessel at the proximal side, and the means may be retracted while engaging the thrombus. Both of the means and inner tube may then be retracted within the outer sheath, which may be in turn subsequently retrieved from the body.

On the one hand, the thrombus removal device according to the present invention employs a pusher shaft having an optimized structure which imparts to the pusher shaft an increased flexibility and hence an increased ability to pass through tortuous portions of a lesion. In addition, the pusher may be provided with a coil and a heat shrink tube, which can enhance force transfer and thus facilitate the release and retraction of the thrombus removal means according to the present invention. On the other hand, the thrombus removal means has an improved structure and employs three-dimensionally contoured, thermally molded inwardly arching ribs extending in a lumen defined by the means, which can facilitate capture of a thrombus, i.e., increasing the likelihood of capturing the thrombus in a single pass, thus reducing the suffering of the patient during the operative produce, and holding the captured thrombus firmly to prevent it from dislodging during the retrieval and travelling in the blood flow to a distal small vessel to cause secondary embolization there. This can reduce the likelihood of complications associated with the thrombus removal.

### BRIEF DESCRIPTION OF THE DRAWINGS

The features and advantages of the present invention will become readily apparent from the following detailed description of embodiments thereof, which is to be read in connection with the accompanying drawings, in which:
FIG. 1 is a schematic illustration of a thrombectomy device;
FIG. 2 schematically illustrates a pusher shaft in an assembled configuration;
FIG. 3 is a schematic illustration of a thrombectomy stent;
FIG. 4 shows an axial view of the thrombectomy stent;
FIG. 5 shows an example of a portion of the thrombectomy stent having inwardly arching ribs distributed as spiraling toward the same direction, in which the portion is shown in an unfolded configuration;
FIG. 6 shows a dimensional relationship between an inwardly arching rib and a cell of a mesh structure;
FIGs. 7 to 12 show other examples of distribution patterns of the inwardly arching ribs, in which, the ribs spiral toward opposite directions in FIG. 7, spiral toward crossing directions in FIGs. 8 and 9, and are distributed in a symmetrical or an alternating manner in FIGs. 10 to 12;
FIG. 13 shows an example of the thrombus removal means fully closed at its distal end;
FIG. 14 shows an example of the thrombus removal means partially closed at the distal end;
FIGs. 15 to 18 show examples of a distal portion of the thrombus removal means, in which the portion is shown in an unfolded configuration.

### DETAILED DESCRIPTION

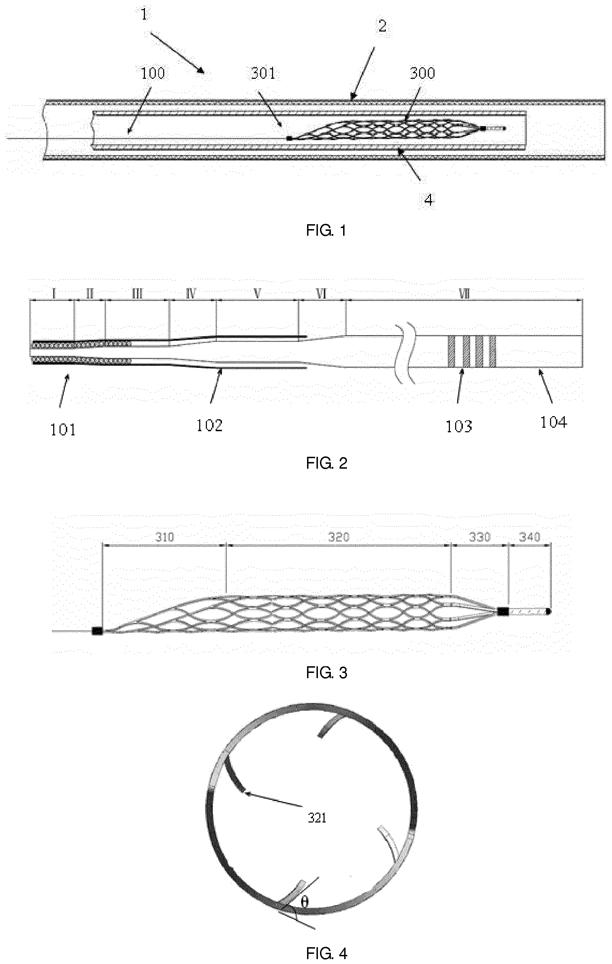

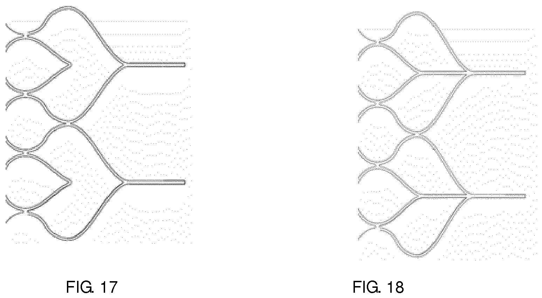

Embodiments of the present invention are described in detail blow with reference to FIGs. 1-18.

For the sake of description, the terms "proximal end" and "distal end" are used in this specification, in which, the term "proximal end" refers to an end located nearer to an operating physician, while the term "distal end" refers to an end located farther from the operating physician.

FIG. 1 shows a thrombus removal device, overall indicated at 1, constructed in accordance with the present invention. The thrombus removal device 1 includes a pusher shaft 100, an outer sheath 2, an inner tube 4 movable relative to the outer sheath, and a thrombus removal means 300. The thrombus removal device 1 also includes a guide wire (not shown) inserted through the inner tube 4 which is in turn received in the outer sheath 2, and the inner tube 4 is movable over the guide wire. The thrombus removal means 300 is attached to the pusher shaft 100 at a portion 301, for example, by soldering, riveting or press fitting. After being so assembled, the pusher shaft 100 and the thrombus removal means 300 are together crimped into the inner tube 4. As well known by those skilled in the art, the thrombus removal means 300 can switch between a contracted configuration and an expanded configuration upon being retracted or advanced by the pusher shaft 100, such that the thrombus removal means 300 is received in the inner tube 4 when in the contracted configuration, and transitions to the expanded configuration after being pushed out of inner tube 4. The outer sheath 2 may also be provided with an occlusion balloon (not shown) at the distal end. The above-described construction is known to those skilled in the art, and therefore further description in this regard is believed unnecessary.

Referring to FIG. 2, the pusher shaft 100 of the thrombus removal device 1 includes a main body 104 made of nitinol or stainless steel. The main body has a generally cylindrical shape with a radius decreasing from a proximal end to a distal end thereof and consisting of multiple sections. In this embodiment, the main body 104 consists of seven sections, in which sections I, III, V and VII have the same radius, and sections II, IV and VI each have a tapered contour. In practice, section VII, indicated at 103, may have a length of 1600-2400 mm and a diameter of 0.3-0.45 mm and may include a mark for providing the physician with an indication about the lengthwise position of the pusher shaft during the operative procedure; section VI has a length of 50-120 mm and a diameter gradually varying from 0.3-0.45 mm to 0.2-0.35 mm; section V has a diameter of 0.2-0.35 mm and a length of 50-120 mm; section IV has a length of 100-200 mm and a diameter gradually varying from 0.2-0.35 mm to 0.15-0.25 mm; section III has a length of 50-120 mm and a diameter of 0.15-0.25 mm; section II has a length of 80-150 mm and a diameter gradually varying from 0.15-0.25 mm to 0.10-0.18 mm; and section I is configured as a cylinder having a length of 25-45 mm and a diameter of 0.10-0.18 mm, or alternatively as a strip having a length of 25-45 mm, a width of 0.10-0.18 mm, and a thickness of 0.04-0.10 mm.

In addition, a length of 25-100 mm of a distal portion of the main body 104 can be wound with a coil 101 which may be made of a platinum, platinum-iridium, silver, etc. wire having a diameter of 0.025-0.070 mm. Further, a 200-600 mm long heat shrink tube 102 may be disposed over the coil. Materials from which the heat shrink tube can be fabricated include PTEF, PVC, PET and FEP. In this embodiment, as shown in FIG. 2, the heat shrink tube 102 spans over sections I-V of the main body 104.

Referring to FIG. 3, the thrombus removal means 300 is overall configured as a mesh structure, for example, by laser-cutting a nitinol tube. In practice, the thrombus removal means 300 has an overall length of 15-60 mm, an outer diameter of 2-6 mm in the expanded configuration, a wall thickness of 0.04-0.12 mm and a strut width of 0.05-0.08 mm. In addition, the thrombus removal means 300 has a proximal portion 310 terminating at an elliptical opening, a closed distal portion 330 terminating at an elongate tip 340, and a tubular intermediate section 320.

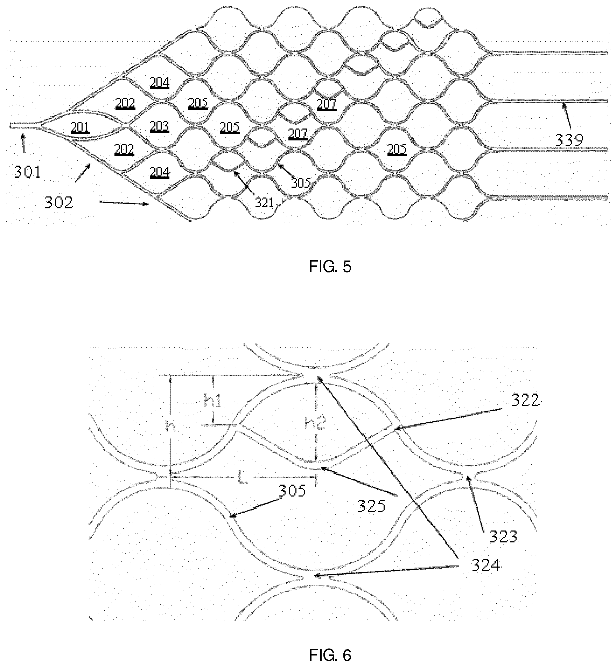

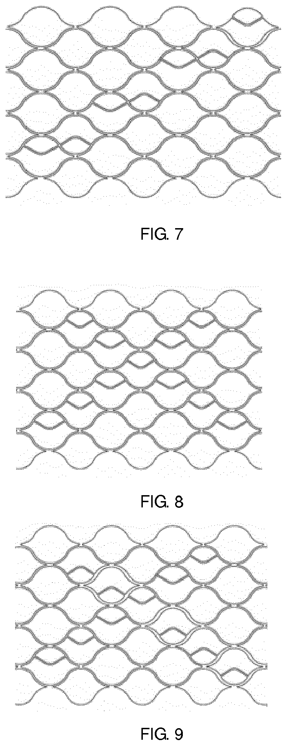

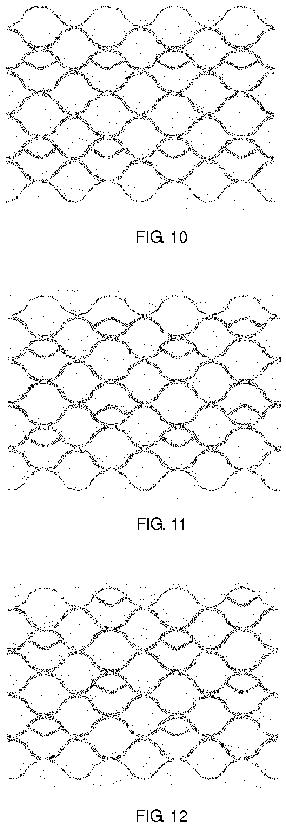

The thrombus removal means 300 defines a lumen. As shown in FIG. 4, the mesh structure of the thrombus removal means 300 includes a plurality of inwardly arching ribs 321 each having a three-dimensional contour, i.e., a non-planar profile. The inwardly arching ribs 321 are formed by heat treatment and integrally formed with the mesh structure in orientations along the axial or circumferential (the circumferential orientation not being part of the invention) direction of the thrombus removal means 300. The inwardly arching ribs 321 may be distributed as spiraling toward the same direction or opposite directions (FIGs. 5 and 7) or intersecting directions (FIGs. 8 and 9), or in a symmetrical or an alternating manner (FIGs. 10, 11 and 12). Additionally, the inwardly arching ribs 321 may be located at the same or different positions with respect to the corresponding cells of the mesh structure and may extend inwardly the same or different lengths.

Each of the inwardly arching rib 321 forms an angle θ with a tangent of an outer circumference of the mesh structure at a position where the inwardly arching rib is attached to the mesh structure. In this embodiment, the angle θ ranges from 30° to 90°. These angles of the inwardly arching ribs 321 may be the same or different, and may gradually increase, decrease or periodically vary along the direction from the proximal end toward the distal end of the thrombus removal means.

FIG. 5 shows the thrombus removal means 300 in an unfolded configuration. As illustrated, the proximal portion 310 includes cells 201, 202, 203 and 204 with different shapes and different areas in the range of 5-12 mm² when not being stressed. The struts 301 at the proximal portion have a length of 0.5-5 mm and a width of 0.05-0.20 mm, and the struts 302 have a width of 0.05-0.15 mm. The intermediate section 320 includes equally sized cells 205 and 207, and the inwardly arching ribs 321 are disposed on the cells 207 with their two ends in fixed connection with struts 305 of the intermediate section 320. The distal portion 330 is composed of several (four in this embodiment) struts 339 with a length of 1-12 mm and a width of 0.03-0.12 mm. The dimensions of the struts 339 may be the same or different.

FIG. 6 shows a cell on which the inwardly arching rib 321 is arranged. The cell is interconnected with adjacent cells at nodes 323 and 324 respectively in the lengthwise and widthwise directions. As illustrated, the cell has a width of 2h and a length of 2L. A distance h1 from a node 322 interconnecting the inwardly arching rib 321 and the mesh structure to a node 324 in the widthwise direction satisfies the relation: 1/4h<h1<3/4h, and a distance h2 from a middle point 325 of the inwardly arching rib 321 to the node 324 satisfies the relation: 3/4h<h2<7/4h. Further, the length and width of the cell satisfies the relation: 1<L/h<2.

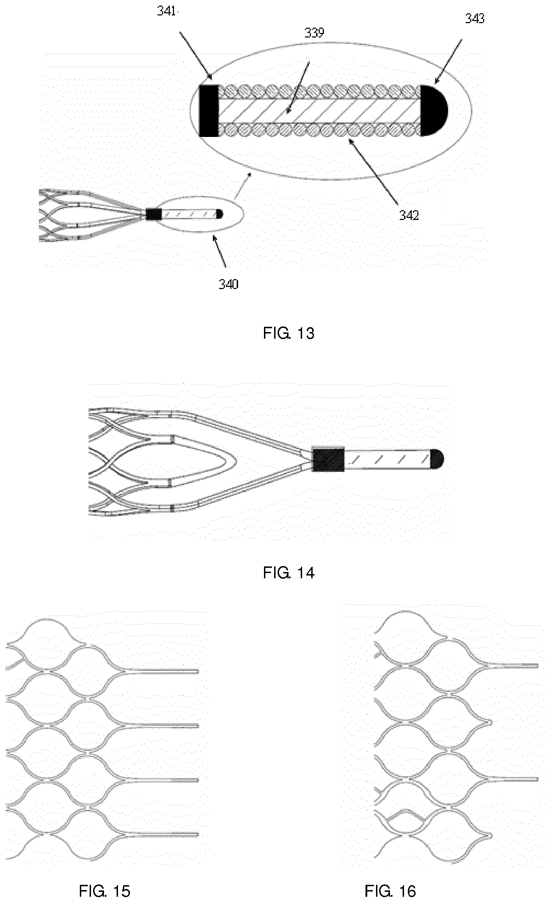

As shown in FIG. 13, the distal portion 330 of the thrombus removal means 300 is closed, and as noted above, the distal portion 330 terminates at the elongate tip 340 which is in connection with the distal portion 330 at an interconnection 341. In this embodiment, a coil 342 is disposed over a strut 339 of the elongate tip 340 and is formed of a platinum, platinum-iridium, silver, etc. wire with a diameter of 0.025-0.070 mm and may have a winding length of 2-10mm. The interconnection 341 may be connected to the coil 342 by soldering, riveting or press fitting, and the metal wire of the coil 342 may be so fused with the strut 339 by laser welding to form a semi-spherical cap 343.

The distal portion 330 of the thrombus removal means 300 may be either fully closed as shown in FIG. 13 or partially closed as shown in FIG. 14. FIGs. 15-18 show examples of the distal portion 330 of the thrombus removal means 300 in an unfolded configuration.

The thrombus removal device 1 according to the present invention may be used in the following manner. In an interventional treatment, an angiographic assessment is first performed to identify an intracranial blood vessel having an occlusive thrombus. The thrombus removal device 1 is then introduced in the vessel via the femoral artery or brachial artery, with the guide wire inserted through the thrombus. The inner tube 4 is then advanced over the guide wire through the thrombus to a desired position with the aid of a radiopaque marker (not shown) on the inner tube 4, with the thrombus removal means 300 being received in the inner tube 4, i.e., in the contracted configuration. Afterward, the guide wire is retrieved, and the thrombus removal means 300 is pushed by the pusher shaft toward a distal end of the inner tube 4. The inner tube 4 is then retracted into the thrombus to deploy the thrombus removal means 300 therein to a desired position by the virtue of a radiopaque marker on the thrombus removal means 300. Because of the shape memory effect of its material, the thrombus removal means 300 switches under the action of the blood temperature to the expanded configuration and pushes against the vessel wall. About 15-20 seconds later, the occlusion balloon of the outer sheath 2 is caused to occlude the vessel at the proximal end, and the thrombus removal means 300 is retracted by the pusher shaft 3 in a state of engaging the thrombus until it is received within the inner tube 4 which is then, in turn, retracted into the outer sheath 2. As a last step of the removal process, the thrombus removal device 1 is wholly retrieved from the human body.

As described above, on the one hand, the thrombus removal device according to the present invention employs a pusher shaft having an optimized structure which imparts, to a leading portion of the pusher shaft, an increased flexibility, torque resistance, force transfer ability and ability to pass through tortuous portions of a lesion, thereby facilitating the penetration of the thrombus removal means according to the present invention through a thrombus, as well as its deployment and retraction. On the other hand, the thrombus removal means has an improved structure and employs three-dimensionally contoured, thermally molded inwardly arching ribs extending in a lumen defined by the thrombus removal means, which can facilitate capture of a thrombus, i.e., increasing the likelihood of capturing the thrombus in a single pass, thus reducing the suffering of the patient during the operative produce, and holding the captured thrombus firmly to prevent it from dislodging during the retrieval and travelling in the blood flow to a distal small vessel to cause secondary embolization there. This can reduce the likelihood of complications associated with the thrombus removal.

While the foregoing embodiments are described in the context of removal of a thrombus from an intracranial vessel, those skilled in the art will realize that the description is merely exemplary. For example, it is to be understood that the means and device of the present invention may also be used in thrombus removal for other vessels with suitable sizes. In addition, the features described in the embodiments disclosed herein may be either used in the manner just as described above, or used individually or in any combination. Conclusively, those skilled in the art can make various changes and modifications to the present invention without departing from the scope thereof.

## Claims

1. A thrombus removal means (300), which is a mesh structure defining a lumen and is switchable between a contracted configuration and an expanded configuration, wherein the thrombus removal means (300) has a plurality of inwardly arching ribs (321) extending from the mesh structure into the lumen, each of the plurality of inwardly arching ribs (321) having a three-dimensional contour and both ends thereof fixed to the mesh structure, the plurality of inwardly arching ribs (321) being oriented in an axial direction of the mesh structure, **characterized in that**:
a distance h1, measured in a widthwise direction, from a location (322) where one of the plurality of inwardly arching ribs (321) is fixed to the mesh structure to a position (324) where a corresponding cell of the mesh structure is interconnected with an adjacent cell thereof in a widthwise direction of the corresponding cell satisfies: 1/4h<h1<3/4h, and/or a distance h2, measured in a widthwise direction, from a middle point (325) of the one of the plurality of inwardly arching ribs (321) to the position (324) where the corresponding cell is interconnected with the adjacent cell satisfies: 3/4h<h2<7/4h, wherein h is half of a width of the corresponding cell,
and wherein one end of said one of the plurality of the inwardly arching ribs (321) is fixed to the corresponding cell at one side of the cell widthwise interconnection position (324), and the other end of the same inwardly arching rib (321) is fixed to the same cell at the other side of the same cell widthwise interconnection position (324).

2. The thrombus removal means (300) according to claim 1, wherein each of the plurality of inwardly arching ribs (321) forms an angle (θ) of 30° to 90° with a tangent of an outer circumference of the mesh structure at a position where the inwardly arching rib is fixed to the mesh structure.

3. The thrombus removal means (300) according to claim 2, wherein the angles formed by the plurality of inwardly arching ribs (321) are identical or not identical, preferably gradually increase, decrease or periodically vary along a direction from a proximal end of the thrombus removal means (300) toward a distal end thereof.

4. The thrombus removal means (300) according to claim 1, wherein the plurality of inwardly arching ribs (321) have one of the following distribution patterns: spiraling toward a same direction, spiraling toward opposite directions, spiraling toward intersecting directions, being distributed in a symmetrical manner, and being distributed in an alternating manner.

5. The thrombus removal means (300) according to claim 1, wherein the mesh structure has cells with a length-to-width ratio greater than 1 and smaller than 2.

6. The thrombus removal means (300) according to claim 1, wherein the thrombus removal means (300) is fully or partially closed at a distal end.

7. The thrombus removal means (300) according to claim 6, wherein, at the distal end, the thrombus removal means terminates at an elongate tip (340) which is wound with a coil (342).

8. A thrombus removal device (1), comprising a guide wire, a pusher shaft (100), an outer sheath (2) and an inner tube (4) movable relative to the outer sheath (2), the guide wire being inserted through the inner tube which is in turn received in the outer sheath and is movable over the guide wire, wherein the thrombus removal device further comprises a thrombus removal means (300) as defined in any of claims 1 to 7, the thrombus removal means (300) being attached to the pusher shaft (3) and being crimped into the inner tube together with the pusher shaft (3), the thrombus removal means (300) being switchable between a contracted configuration and an expanded configuration upon being retracted or advanced by the pusher shaft (3) such that the thrombus removal means (300) is in the contracted configuration when received in the inner tube and transitions to the expanded configuration after being pushed out of the inner tube.

9. The thrombus removal device according to claim 8, wherein the outer sheath comprises an occlusion balloon disposed at a distal end of the outer sheath.

10. The thrombus removal device according to claim 8, wherein the thrombus removal means (300) is attached to the pusher shaft by means of a tethering connector which is positioned closely adjacent to a vessel wall when the thrombus removal means (300) is in the expanded configuration.

11. The thrombus removal device according to any of claims 8 to 9, wherein the pusher shaft has a radius decreasing stepwise from a proximal end of the pusher shaft toward a distal end thereof.

12. The thrombus removal device according to claim 11, wherein the pusher shaft has a distal portion wound with a coil (342).

## Patentansprüche

1. Thrombusentfernungsmittel (300), wobei es sich um eine Netzstruktur handelt, die ein Lumen definiert und zwischen einer zusammengezogenen Konfiguration und einer auseinandergezogenen Konfiguration gewechselt werden kann, wobei das Thrombusentfernungsmittel (300) eine Mehrzahl von sich nach innen wölbenden Rippen (321) umfasst, die sich von der Netzstruktur in das Lumen erstrecken, wobei jede der Mehrzahl von sich nach innen wölbenden Rippen (321) eine dreidimensionale Kontur aufweist und beide Enden davon an der Netzstruktur fixiert sind, die Mehrzahl von sich nach innen wölbenden Rippen (321) in einer axialen Richtung der Netzstruktur orientiert ist, **dadurch gekennzeichnet, dass**:
ein Abstand h1 gemessen in einer Breitenrichtung von einer Stelle (322), an der eine der Mehrzahl von sich nach innen wölbenden Rippen (321) an der Netzstruktur fixiert ist, zu einer Position (324), in der eine entsprechende Zelle der Netzstruktur mit einer benachbarten Zelle davon in einer Breitenrichtung der entsprechenden Zelle verbunden ist, erfüllt: 1/4 h < h1 < 3/4 h, und/oder ein Abstand h2 gemessen in einer Breitenrichtung von einem Mittelpunkt (325) der einen der Mehrzahl von sich nach innen wölbenden Rippen (321) zu der Position (324), in der die entsprechende Zelle mit der benachbarten Zelle verbunden ist, erfüllt: 3/4 h < h2 < 7/4 h, wobei h die Hälfte einer Breite der entsprechenden Zelle ist, und wobei ein Ende der einen der Mehrzahl der sich nach innen wölbenden Rippen (321) an der entsprechenden Zelle auf einer Seite der Verbindungsposition (324) der Zellenbreite fixiert ist, und das andere Ende der gleichen sich nach innen wölbenden Rippe (321) an der gleichen Zelle auf der anderen Seite der gleichen Verbindungsposition (324) der Zellenbreite fixiert ist.

2. Thrombusentfernungsmittel (300) nach Anspruch 1, wobei jede der Mehrzahl von sich nach innen wölbenden Rippen (321) mit einer Tangente eines Außenumfangs der Netzstruktur in einer Position, in der die sich nach innen wölbende Rippe an der Netzstruktur fixiert ist, einen Winkel (θ) von 30° bis 90° bildet.

3. Thrombusentfernungsmittel (300) nach Anspruch 2, wobei die Winkel, die durch die Mehrzahl von sich nach innen wölbenden Rippen (321) gebildet werden, identisch oder nicht identisch sind, vorzugsweise allmählich zunehmen, abnehmen oder periodisch variieren entlang einer Richtung von einem proximalen Ende des Thrombusentfernungsmittels (300) zu einem distalen Ende davon.

4. Thrombusentfernungsmittel (300) nach Anspruch 1, wobei die Mehrzahl von sich nach innen wölbenden Rippen (321) eines der folgenden Verteilungsmuster aufweist: spiralförmig in einer gleichen Richtung, spiralförmig in entgegengesetzten Richtungen, spiralförmig in überkreuzenden Richtungen, symmetrisch verteilt und alternierend verteilt.

5. Thrombusentfernungsmittel (300) nach Anspruch 1, wobei die Netzstruktur Zellen mit einem Verhältnis von Länge zu Breite aufweist, das größer als 1 und kleiner als 2 ist.

6. Thrombusentfernungsmittel (300) nach Anspruch 1, wobei das Thrombusentfernungsmittel (300) an einem distalen Ende ganz oder teilweise geschlossen ist.

7. Thrombusentfernungsmittel (300) nach Anspruch 6, wobei das Thrombusentfernungsmittel am distalen Ende in einer länglichen Spitze (340) endet, die mit einer Spule (342) umwickelt ist.

8. Thrombusentfernungsvorrichtung (1), umfassend einen Führungsdraht, einen Schiebeschaft (100), einen Außenmantel (2) und ein Innenrohr (4), das in Bezug auf den Außenmantel (2) beweglich ist, wobei der Führungsdraht durch das Innenrohr eingeführt wird, das wiederum im Außenmantel aufgenommen wird und über den Führungsdraht bewegt werden kann, wobei die Thrombusentfernungsvorrichtung ferner ein Thrombusentfernungsmittel (300) nach einem der Ansprüche 1 bis 7 umfasst, das Thrombusentfernungsmittel (300) am Schiebeschaft (3) befestigt und zusammen mit dem Schiebeschaft (3) in das Innenrohr gecrimpt wird, das Thrombusentfernungsmittel (300) bei Zurückziehen oder Vorschieben durch den Schiebeschaft (3) zwischen einer zusammengezogenen Konfiguration und einer auseinandergezogenen Konfiguration gewechselt werden kann, derart dass das Thrombusentfernungsmittel (300) in der zusammengezogenen Konfiguration ist, wenn im Innenrohr aufgenommen, und in die auseinandergezogene Konfiguration übergeht, nachdem es aus dem Innenrohr geschoben wird.

9. Thrombusentfernungsvorrichtung (1) nach Anspruch 8, wobei der Außenmantel einen Verschlussballon umfasst, der an einem distalen Ende des Außenmantels angeordnet ist.

10. Thrombusentfernungsvorrichtung (1) nach Anspruch 8, wobei das Thrombusentfernungsmittel (300) durch einen Bindeglied am Schiebeschaft befestigt ist, das unmittelbar benachbart zu einer Gefäßwand positioniert ist, wenn das Thrombusentfernungsmittel (300) in der auseinandergezogenen Konfiguration ist.

11. Thrombusentfernungsvorrichtung (1) nach einem der Ansprüche 8 bis 9, wobei der Schiebeschaft einen Radius aufweist, der von einem proximalen Ende des Schiebeschafts zu einem distalen Ende davon schrittweise abnimmt.

12. Thrombusentfernungsvorrichtung (1) nach Anspruch 11, wobei der Schiebeschaft ein distales Ende aufweist, das mit einer Spule (342) umwickelt ist.

## Revendications

1. Moyen de retrait de thrombus (300), qui est une structure maillée définissant une lumière et qui peut passer entre une configuration contractée et une configuration déployée, dans lequel le moyen de retrait de thrombus (300) possède une pluralité de nervures courbées vers l'intérieur (321) s'étendant depuis la structure maillée dans la lumière, chacune de la pluralité de nervures courbées vers l'intérieur (321) ayant un contour en trois dimensions et ses deux extrémités étant fixées sur la structure maillée, la pluralité de nervures courbées vers l'intérieur (321) étant orientée dans une direction axiale de la structure maillée, **caractérisé en ce que** :
une distance h1, mesurée dans le sens de la largeur, entre un emplacement (322) auquel l'une de la pluralité de nervures courbées vers l'intérieur (321) est fixée sur la structure maillée et un emplacement (324) auquel une cellule correspondante de la structure maillée est reliée à une cellule adjacente de celle-ci dans le sens de la largeur de la cellule correspondante satisfait : 1/4h < h1< 3/4h, et/ou une distance h2, mesurée dans le sens de la largeur, entre un point médian (325) de l'une de la pluralité de nervures courbées vers l'intérieur (321) et l'emplacement (324) auquel la cellule correspondante est reliée à la cellule adjacente satisfait : 3/4h < h2 < 7/4h , où h est la moitié d'une largeur de la cellule correspondante, et dans lequel une extrémité de l'une de ladite pluralité de nervures courbées vers l'intérieur (321) est fixée sur la cellule correspondante au niveau d'un côté de l'emplacement de liaison dans le sens de la largeur de la cellule (324), et l'autre extrémité de la même nervure courbée vers l'intérieur (321) est fixée sur la même cellule au niveau de l'autre côté du même emplacement de liaison dans le sens de la largeur de la cellule (324).

2. Moyen de retrait de thrombus (300) selon la revendication 1, dans lequel chacune de la pluralité de nervures courbées vers l'intérieur (321) forme un angle (θ) de 30° à 90° avec une tangente d'une circonférence externe de la structure maillée à un emplacement auquel la nervure courbée vers l'intérieur est fixée sur la structure maillée.

3. Moyen de retrait de thrombus (300) selon la revendication 2, dans lequel les angles formés par la pluralité de nervures courbées vers l'intérieur (321) sont identiques ou non identiques, augmentent de préférence progressivement, diminuent ou varient périodiquement le long d'une direction entre une extrémité proximale du moyen de retrait de thrombus (300) et une extrémité distale de celui-ci.

4. Moyen de retrait de thrombus (300) selon la revendication 1, dans lequel la pluralité de nervures courbées vers l'intérieur (321) possède l'un des modèles de répartition suivants : en spirale dans une même direction, en spirale dans des directions opposées, en spirale dans des directions croisées, de manière symétrique, et de manière alternée.

5. Moyen de retrait de thrombus (300) selon la revendication 1, dans lequel la structure maillée possède des cellules avec un rapport longueur-largeur supérieur à 1 et inférieur à 2.

6. Moyen de retrait de thrombus (300) selon la revendication 1, dans lequel le moyen de retrait de thrombus (300) est complètement ou partiellement fermé au niveau d'une extrémité distale.

7. Moyen de retrait de thrombus (300) selon la revendication 6, dans lequel, au niveau de l'extrémité distale, le moyen de retrait de thrombus se termine au niveau d'une extrémité allongée (340) qui est enroulée avec une bobine (342).

8. Dispositif de retrait de thrombus (1), comprenant un fil de guidage, un arbre de poussée (100), une gaine externe (2) et un tube interne (4) déplaçable par rapport à la gaine externe (2), le fil de guidage étant inséré dans le tube interne qui est à son tour reçu dans la gaine externe et peut se déplacer sur le fil de guidage, dans lequel le dispositif de retrait de thrombus comprend en outre un moyen de retrait de thrombus (300) selon l'une quelconque des revendications 1 à 7, le moyen de retrait de thrombus (300) étant fixé sur l'arbre pousseur (3) et étant serti dans le tube interne avec l'arbre de poussée (3), le moyen de retrait de thrombus (300) pouvant passer d'une configuration contractée à une configuration déployée lorsqu'il est rétracté ou avancé par l'arbre de poussée (3) de sorte que le moyen de retrait de thrombus (300) se trouve dans la configuration contractée lorsqu'il est reçu dans le tube interne et passe à la configuration déployée après avoir été sorti du tube interne.

9. Dispositif de retrait de thrombus selon la revendication 8, dans lequel la gaine externe comprend un ballonnet d'occlusion disposé au niveau d'une extrémité distale de la gaine externe.

10. Dispositif de retrait de thrombus selon la revendication 8, dans lequel le moyen de retrait de thrombus (300) est fixé sur l'arbre de poussée au moyen d'un connecteur d'attache qui est positionné de manière étroitement adjacente à une paroi de vaisseau lorsque le moyen de retrait de thrombus (300) se trouve dans la configuration déployée.

11. Dispositif de retrait de thrombus selon l'une quelconque des revendications 8 à 9, dans lequel l'arbre de poussée possède un rayon qui diminue progressivement d'une extrémité proximale de l'arbre de poussée vers une extrémité distale de celui-ci.

12. Dispositif de retrait de thrombus selon la revendication 11, dans lequel l'arbre de poussée possède une partie distale enroulée avec une bobine (342).
